# EUROPEAN PATENT APPLICATION

(11) **EP 4 667 476 A1**
(43) Date of publication of application: **24.12.2025**
(21) Application number: 24757119.3
(22) Date of filing: 07.02.2024
(51) Int. Cl.: C07F 9/54, A61K 31/662, A61P 35/00, A61P 37/00

(54) **NOVEL MITOCHONDRIA-TARGETING COMPOUNDS**

(30) Priority: 13.02.2023 KR 20230018673
(71) Applicant: THE CATHOLIC UNIVERSITY OF KOREA INDUSTRY-ACADEMIC COOPERATION FOUNDATION, Seoul 06591 (KR)
(72) Inventor: PARK, Joon-Hyuck, Seoul 08725 (KR); CHO, Mi-La, Seoul 04427 (KR); SHIN, Jeong-Su, Seoul 01776 (KR); SONG, Kyo-Young, Seoul 06597 (KR); JHUN, Joo-Yeon, Seoul 07993 (KR); LEE, Seung-Yoon, Seoul 06954 (KR); YANG, Seung-Cheon, Seoul 08735 (KR)
(74) Representative: Isarpatent
(86) International application number: PCT/KR2024/001816
(87) International publication number: WO 2024/172390

(57) **Abstract**

The present disclosure relates to a technique for synthesizing with high yield a novel compound targeting mitochondria through a simple process, and the novel compound synthesized thereby. The novel compound targeting mitochondria of the present disclosure can be synthesized with high yield through a simple process and the compound thus prepared and nanoparticles containing same exhibit the therapeutic effects of targeting mitochondria, inhibiting gastric cancer, and treating inflammatory bowel disease, and systemic sclerosis and thus can be utilized for mitochondrial targeting applications, as anticancer compositions, or as therapeutic compositions for immune diseases.

## Description

### [Technical Field]

The present disclosure relates to a technique for synthesizing with high yield a novel compound targeting mitochondria through a simple process, and the novel compound synthesized by the technique.

### [Background Art]

Cancer is a condition in which the proliferation and death of cells are not normally controlled by various changes in gene expression, and the abnormal growth of cells is caused, and infiltrates and destroys adjacent tissues, metastasizes to other regions, and eventually leads to death. The cause of cancer, that is, a mechanism through which normal cells are transformed into cancer cells, has not been precisely identified, but as far as it is concerned, it has been known that external factors such as environmental factors, chemicals, radiation, and viruses, internal factors such as genetic factors and immunological factors, and the like are complicatedly involved to cause the cancer. The cancer is largely classified into blood cancer showing abnormality in the number of blood cells and solid cancer in the form of a mass of cells having predetermined hardness and shape in the body. The cancer may occur in almost all parts of the body, such as blood and tissue, and examples thereof may include lung cancer, gastric cancer, breast cancer, oral cancer, liver cancer, uterine cancer, esophageal cancer, skin cancer, and the like. In the treatment of cancer, the main methods include most surgery or radiation therapy and chemotherapy using chemotherapeutic agents that inhibit cell proliferation. Among these, gastric cancer (GC) is known to be the fifth most common malignant tumor among various cancers worldwide and the fourth leading cause of death caused by cancer.

Meanwhile, diseases caused by immune hypersensitivity are increasing worldwide, but the fundamental causes of occurrence of these diseases have not been sufficiently identified. A current therapeutic method for diseases caused by excessive immune responses is to alleviate or reduce various symptoms caused by the diseases by administering immunosuppressants alone or in combination. The immunosuppressants refer to various materials used to reduce or block the ability (humoral immune response) to produce antibodies by a host or the ability to cause a cellular immune response, with respect to the action of an antigen. These immunosuppressants may be effectively used not only in the field of organ transplantation, but also in autoimmune diseases such as lupus and rheumatoid arthritis, and skin hypersensitivity such as atopy and allergies. An excellent immunosuppressant needs to be able to control the imbalance of the immune responses, ensure safety for the human body, and have a low incidence of disease recurrence during long-term treatment.

Currently used immunosuppressants include cyclosporin A, FK506, and the like, which are natural product-derived compounds with complex chemical structures, and are uneconomical due to a problem of high cost in terms of raw material supply, so that there is a risk of causing various side effects due to long-term administration. Accordingly, there is an urgent need for the development of new immunosuppressants that are able to be economically produced with low toxicity and immune tolerance induction. In relation to immunity, T cells correspond to one of cell groups that play a central role in the immune system as a biological defense system against various pathogens. The T cells are produced in the thymus of the human body and differentiate into T cells with unique characteristics through a series of differentiation processes. The differentiated T cells are largely divided into type 1 helper cells (Th1) and type 2 helper cells (Th2) depending on the function. Among these, the main function of Th1 cells is involved in cell-mediated immunity, and Th2 cells are involved in humoral immunity. In the immune system, these two cell groups check each other so as to be overactivated, thereby maintaining the balance of the immune system. Therefore, it can be seen that most immune diseases are caused by the imbalance between the two immune cells, and for example, it is known that when the activity of Th1 cells increases abnormally, autoimmune diseases may occur, and when the activity of Th2 cells increases abnormally, immune diseases caused by hypersensitivity may occur. In addition, there are known a new group called immunoregulatory T cells (Tregs) capable of regulating the activity of Th1 cells and another group called Th17 cells that are produced during a differentiation process of T cells. It has been found that, unlike Treg cells, Th17 cells are involved in the forefront of the inflammatory response shown in immune diseases, thereby maximizing signals of the inflammatory response and accelerating the progression of the diseases.

Therefore, in the case of autoimmune diseases that are not controlled by Treg cells among the autoimmune diseases, the development of therapeutic agents for autoimmune diseases targeting the inhibition of Th17 cell activity has received significant attention.

### [Disclosure]

### [Technical Problem]

An object of the present disclosure is to provide a compound targeting mitochondria.

Another object of the present invention is to provide a mitochondria-targeting composition.

Yet another object of the present disclosure is to provide a pharmaceutical composition for preventing or treating diseases associated with mitochondrial dysfunction.

Still another object of the present disclosure is to provide a food composition for preventing or improving diseases associated with mitochondrial dysfunction.

Still another object of the present disclosure is to provide a health functional food composition for preventing or improving diseases associated with mitochondrial dysfunction.

Still another object of the present disclosure is to provide a method for preparing a compound targeting mitochondria.

Still another object of the present disclosure is to provide nanoparticles including a compound targeting mitochondria.

Still another object of the present disclosure is to provide a pharmaceutical composition for preventing or treating immune diseases.

Still another object of the present disclosure is to provide a food composition for preventing or improving immune diseases.

Still another object of the present disclosure is to provide a health functional food composition for preventing or improving immune diseases.

Still another object of the present disclosure is to provide a method for preventing or treating diseases associated with mitochondrial dysfunction.

Still another object of the present disclosure is to provide a method for preventing or treating immune diseases.

### [Technical Solution]

To achieve the object, the present disclosure provides a compound represented by the following Chemical Formula 1, a hydrate thereof, a solvate thereof, or a pharmaceutically acceptable salt thereof:

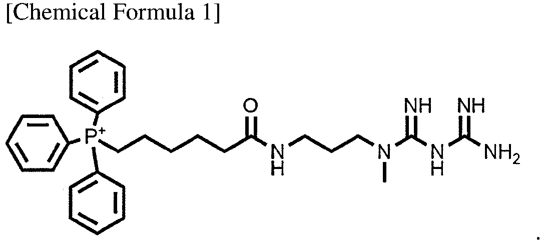

In addition, to achieve another object, the present disclosure provides a mitochondria-targeting composition including the compound of the present disclosure, a hydrate thereof, a solvate thereof, or a pharmaceutically acceptable salt thereof.

In addition, to achieve yet another object, the present disclosure provides a pharmaceutical composition for preventing or treating diseases associated with mitochondrial dysfunction, including the compound of the present disclosure, a hydrate thereof, a solvate thereof, or a pharmaceutically acceptable salt thereof.

Further, the present disclosure provides a pharmaceutical composition for preventing or treating diseases associated with mitochondrial dysfunction, including nanoparticles including the compound of the present disclosure, a hydrate thereof, a solvate thereof, or a pharmaceutically acceptable salt thereof.

In addition, to achieve yet another object, the present disclosure provides a food composition for preventing or improving diseases associated with mitochondrial dysfunction, including the compound of the present disclosure, a hydrate thereof, a solvate thereof, or a pharmaceutically acceptable salt thereof.

Further, the present disclosure provides a food composition for preventing or treating diseases associated with mitochondrial dysfunction, including nanoparticles including the compound of the present disclosure, a hydrate thereof, a solvate thereof, or a pharmaceutically acceptable salt thereof.

In addition, to achieve still another object, the present disclosure provides a health functional food composition for preventing or improving diseases associated with mitochondrial dysfunction, including the compound of the present disclosure, a hydrate thereof, a solvate thereof, or a pharmaceutically acceptable salt thereof.

Further, the present disclosure provides a health functional food composition for preventing or treating diseases associated with mitochondrial dysfunction, including nanoparticles including the compound of the present disclosure, a hydrate thereof, a solvate thereof, or a pharmaceutically acceptable salt thereof.

In addition, to achieve still another object, the present disclosure provides a method for preparing a compound targeting mitochondria according to the present disclosure.

In addition, to achieve still another object, the present disclosure provides nanoparticles including the compound of the present disclosure, a hydrate thereof, a solvate thereof, or a pharmaceutically acceptable salt thereof.

In addition, to achieve still another object, the present disclosure provides a pharmaceutical composition for preventing or treating immune diseases, including the compound of the present disclosure, a hydrate thereof, a solvate thereof, or a pharmaceutically acceptable salt thereof.

Further, the present disclosure provides a pharmaceutical composition for preventing or treating immune diseases, including nanoparticles including the compound of the present disclosure, a hydrate thereof, a solvate thereof, or a pharmaceutically acceptable salt thereof.

In addition, to achieve still another object, the present disclosure provides a food composition for preventing or improving immune diseases, including the compound of the present disclosure, a hydrate thereof, a solvate thereof, or a pharmaceutically acceptable salt thereof.

Further, the present disclosure provides a food composition for preventing or improving immune diseases, including nanoparticles including the compound of the present disclosure, a hydrate thereof, a solvate thereof, or a pharmaceutically acceptable salt thereof.

In addition, to achieve still another object, the present disclosure provides a health functional food composition for preventing or improving immune diseases, including the compound of the present disclosure, a hydrate thereof, a solvate thereof, or a pharmaceutically acceptable salt thereof.

Further, the present disclosure provides a health functional food composition for preventing or improving immune diseases, including nanoparticles including the compound of the present disclosure, a hydrate thereof, a solvate thereof, or a pharmaceutically acceptable salt thereof.

In addition, to achieve still another object, the present disclosure provides a method for preventing or treating diseases associated with mitochondrial dysfunction, including administering, to a subject, the compound of the present disclosure, a hydrate thereof, a solvate thereof, or a pharmaceutically acceptable salt thereof.

Further, the present disclosure provides a method for preventing or treating diseases associated with mitochondrial dysfunction, including administering, to a subject, nanoparticles including the compound of the present disclosure, a hydrate thereof, a solvate thereof, or a pharmaceutically acceptable salt thereof.

In addition, to achieve still another object, the present disclosure provides a method for preventing or treating immune diseases, including administering, to a subject, the compound of the present disclosure, a hydrate thereof, a solvate thereof, or a pharmaceutically acceptable salt thereof.

Further, the present disclosure provides a method for preventing or treating immune diseases, including administering, to a subject, nanoparticles including the compound of the present disclosure, a hydrate thereof, a solvate thereof, or a pharmaceutically acceptable salt thereof.

### [Advantageous Effects]

According to the present disclosure, the novel compound targeting mitochondria can be synthesized with high yield through a simple process and the compound thus prepared and nanoparticles containing the compound exhibit the therapeutic effects of targeting mitochondria, inhibiting gastric cancer, and treating inflammatory bowel disease and systemic sclerosis, and thus can be utilized for mitochondrial targeting applications, as anticancer compositions, or as therapeutic compositions for immune diseases.

### [Description of Drawings]

FIG. 1 is a schematic diagram illustrating the synthesis of JH-001, a mitochondria-targeting compound according to the present disclosure.
FIGS. 2 and 3 are diagrams showing mass analysis results of JH-001, a mitochondria-targeting compound.
FIG. 4 is a diagram showing NMR analysis results of JH-001, a mitochondria-targeting compound.
FIG. 5 is a diagram showing an effect of inhibiting gastric cancer cell proliferation by treatment with JH-001, a mitochondria-targeting compound.
FIG. 6 is a diagram comparing effects of inhibiting gastric cancer cell proliferation by treatment with the same concentration of JH-001, a mitochondria-targeting compound, or Metformin which is a conventional known mitochondrial targeting drug.
FIG. 7 is a diagram showing an effect of inhibiting PD-L1 expression in gastric cancer cells by treatment with JH-001, a mitochondria-targeting compound.
FIG. 8 is a diagram comparing effects of inhibiting PD-L1 expression in gastric cancer cells by treatment with the same concentration of JH-001, a mitochondria-targeting compound, or Metformin, a conventional known mitochondrial targeting drug.
FIG. 9 is a diagram showing an effect of restoring a colon length in a DSS-induced colitis disease model by administration of JH-001, a mitochondria-targeting compound.
FIG. 10 is a diagram showing an effect of inhibiting the expression of Th1, Th2, and Th17 in a DSS-induced colitis disease model by administration of JH-001, a mitochondria-targeting compound.
FIG. 11 shows absorbance measured after incorporating JH-001, a mitochondria-targeting compound, into gold nanoparticles.
FIG. 12 is a histogram of a hydrated size distribution after incorporating JH-001, a mitochondria-targeting compound, into gold nanoparticles, which is a diagram confirming the size.
FIG. 13 is a transmission electron microscope image after incorporating JH-001, a mitochondria-targeting compound, into gold nanoparticles, which is a diagram confirming the size. In FIG. 13, a bar at the lower right indicates 100 nm.
FIG. 14 is a schematic diagram showing a process for fabricating a vimentin-sensitized animal model to confirm an effect of JH-001, a mitochondria-targeting compound, on systemic sclerosis.
FIG. 15 is a diagram showing results of ELISA analysis of vimentin antigen-specific IgG and IgG1 in the serum of a systemic sclerosis animal model by treatment with JH-001, a mitochondria-targeting compound. FIG. 15A shows a result of IgG quantification, and FIG. 15B shows a result of IgG1 quantification.
FIG. 16 is a diagram showing skin fibrosis in a systemic sclerosis animal model by treatment with JH-001, a mitochondria-targeting compound, using H&E and MT staining. FIG. 16A shows a staining result, and FIG. 16B shows a result of quantifying the staining result.

### [Best Mode]

Hereinafter, embodiments of the present disclosure will be described in detail with reference to the accompanying drawings. However, the following embodiments are presented as examples for the present disclosure, and when it is determined that the detailed description of well-known technologies or configurations known to those skilled in the art may unnecessarily obscure the gist of the present disclosure, the detailed description thereof may be omitted, and the present disclosure is not limited thereto. Various modifications and applications of the present disclosure are possible within the description of claims to be described below and the equivalent scope interpreted therefrom.

In addition, terminologies used herein are terminologies used to properly express preferred embodiments of the present disclosure, which may vary according to a user, an operator's intention, or customs in the art to which the present disclosure pertains. Therefore, these terminologies used herein will be defined based on the contents throughout the specification. Throughout this specification, unless explicitly described to the contrary, when a certain part "comprises" a certain component, it will be understood to imply the inclusion of stated elements, not the exclusion of any other elements.

All technical terms used in the present disclosure, unless otherwise defined, are used as the meaning as commonly understood by those skilled in the related art of the present disclosure. In addition, although preferred methods and samples are described herein, similar or equivalent methods and samples thereto are also included in the scope of the present disclosure. The contents of all publications described herein as references are incorporated in the present disclosure.

In one aspect, the present disclosure relates to a compound represented by the following Chemical Formula 1, a hydrate thereof, a solvate thereof, or a pharmaceutically acceptable salt thereof:

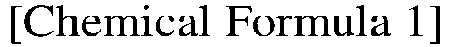

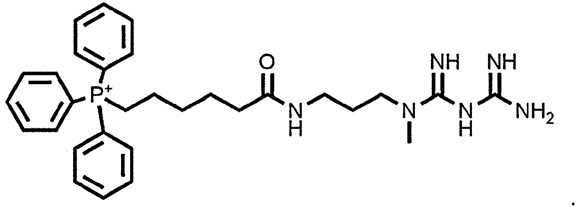

In one embodiment, the compound represented by Chemical Formula 1 may be prepared by reacting (5-carboxypentyl)triphenylphosphonium bromide with carbonyldiimidazole to obtain a compound represented by the following Chemical Formula 2, adding N-methyl-1,3-propanediamine dropwise to the compound represented by Chemical Formula 2 and mixing and reacting the mixture to obtain a compound represented by the following Chemical Formula 3, and then reacting the compound represented by Chemical Formula 3 with dicyandiamide: 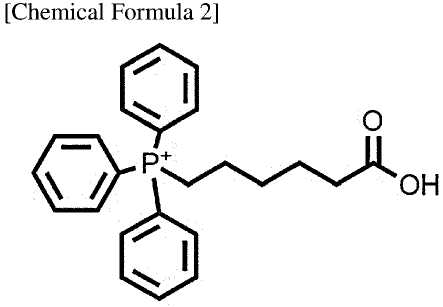 ; and

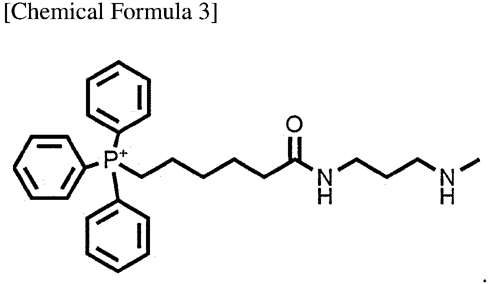

In one embodiment, the compound of the present disclosure, the hydrate thereof, the solvate thereof, or the pharmaceutically acceptable salt thereof may be used for targeting mitochondria.

In one embodiment, the compound represented by Chemical Formula 1 of the present disclosure may be used in the form of a pharmaceutically acceptable salt, and as the salt, an acid addition salt formed by a pharmaceutically acceptable free acid is useful. The acid addition salt is obtained from inorganic acids such as hydrochloric acid, nitric acid, phosphoric acid, sulfuric acid, hydrobromic acid, hydroiodic acid, nitrous acid or phosphorous acid, and non-toxic organic acids such as aliphatic mono- and di-carboxylates, phenyl-substituted alkanoates, hydroxy alkanoates and alkanedioates, aromatic acids, and aliphatic and aromatic sulfonic acids. These pharmaceutically non-toxic salts include sulfate, pyrosulfate, bisulfate, sulfite, bisulfite, nitrate, phosphate, monohydrogen phosphate, dihydrogen phosphate, metaphosphate, pyrophosphate chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexane-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutyrate, citrate, lactate, hydroxybutyrate, glycolate, malate, tartrate, methanesulfonate, propanesulfonate, naphthalene-1-sulfonate, naphthalene-2-sulfonate or mandelate.

The acid addition salts according to the present disclosure may be prepared by a conventional method, for example, by dissolving the compound in an excess acidic aqueous solution and precipitating the salt using a water-miscible organic solvent, such as methanol, ethanol, acetone or acetonitrile. In addition, the acid addition salts may also be prepared by evaporating and drying a solvent or excess acid from the mixture or by suction-filtering the precipitated salt. In addition, pharmaceutically acceptable metal salts may be prepared using bases. Alkali metal or alkaline earth metal salts may be obtained, for example, by dissolving the compound in an excess alkali metal hydroxide or alkaline earth metal hydroxide solution, filtering a non-dissolved compound salt, and then evaporating and drying a filtrate. At this time, as the metal salt, it is pharmaceutically suitable to prepare a sodium, potassium or calcium salt. Further, silver salts corresponding thereto may be obtained by reacting the alkali metal or alkaline earth metal salt with a suitable silver salt (e.g., silver nitrate).

In one embodiment, the compound of the present disclosure, the hydrate thereof, the solvate thereof, or the pharmaceutically acceptable salt thereof may further include a labeling material. The labeling material may be at least one selected from the group consisting of a chromogenic enzyme, a radioisotope, a chromophore, a chromogenic material, a luminescent material, a fluorescer, superparamagnetic particles, and ultrasuper paramagnetic particles, but is not limited thereto.

In one embodiment, the chromogenic enzyme may be at least any one selected from the group consisting of streptavidin-conjugated horseradish peroxidase (HRP), alkaline phosphatase (AP), β-galactosidase, luciferase, acetylcholinesterase, urease, catalase, asparginase, ribonuclease, malate dehydrogenase, staphylococcal nuclease, triose phosphate isomerase, glucose oxidase, cytochrome P450, and peroxidase compounds, but is not limited thereto.

In one embodiment, the fluorescer may be a fluorescent protein, a photoprotein, luciferase, a fluorescent dye or a time-resolved fluorescence (TRF), and the fluorescer may be at least any one selected from the group consisting of Alexa Fluor 350, 405, 430, 488, 500, 514, 633, 647, 660, 680, 700, cy3, cy5, cy7, rubpy(tris(2,2-bipyridyl)ruthenium(II)), fluoresein Isothiocyanate (FITC), rhodamine 6G, rhodamine B, 5(6)-carboxytetramethylrhodamine (TAMRA), Texas Red, 4,6-diamidino-2-phenylindole (DAPI) and coumarin, but is not limited thereto. Preferably, the fluorescer may be TAMRA.

In an aspect, the present disclosure relates to a mitochondria-targeting composition, including the compound represented by Chemical Formula 1 of the present disclosure, a hydrate thereof, a solvate thereof, or a pharmaceutically acceptable salt thereof.

In one embodiment, the targeting composition may further include one or more selected from a solvent, an acid, a base, and a buffer solution. The targeting composition may be prepared by adding the above-described compound to a solvent, a buffer solution, or a mixture thereof, and adding an acid or a base thereto. In addition, the targeting composition may further include other additives that may be used in the art. The contents of the solvent, the acid, the base, and the buffer solution included in the composition may be appropriately adjusted depending on the required performance. The solvent may include water, tetrahydrofuran (THF), methanol, ethanol, an HI aqueous solution, N,N-dimethylformamide (DMF), or a combination thereof. Alternatively, the composition may include a combination with other drugs and chemical molecules. The corresponding drug may include a drug having an effect on a specific region, but is not limited thereto, and may include any drug which may be used as a chemical sample, such as a drug, a contrast agent, a nutritional supplement, or a fluorescent material.

As used herein, the term "targeting" refers to the ability to target a specific cell or an *in vivo* region, and means the characteristic of a material to be specifically delivered to the corresponding region when distributed in the body. The targeting characteristic is a characteristic that appears only in a specific *in vivo* part, and may mean a characteristic in which a significantly larger amount is delivered than the remaining parts excluding the corresponding part.

In one aspect, the present disclosure provides a pharmaceutical composition for preventing or treating diseases associated with mitochondrial dysfunction, including the compound of the present disclosure, a hydrate thereof, a solvate thereof, or a pharmaceutically acceptable salt thereof.

In one embodiment, the disease associated with mitochondrial dysfunction may be at least any one selected from the group consisting of cancer disease, ischemic brain disease, ischemic heart disease, stroke, migraine, anemia, Parkinson's disease, Alzheimer's disease, amyotrophic lateral sclerosis (ALS), mitochondrial encephalomyopathy, liver cirrhosis, Fanconi syndrome, atypical phenylketonuria, asthma, diabetes, arteriosclerosis, hypertension, hypercholesterolemia, optic nerve disease, obesity, and depression, but is not limited thereto.

In an embodiment, the cancer may be cancer caused by mitochondrial hypofunction, and may be at least any one selected from the group consisting of gastric cancer, brain tumor, melanoma, myeloma, non-small cell lung cancer, oral cancer, liver cancer, colon cancer, breast cancer, lung cancer, bone cancer, pancreatic cancer, skin cancer, head or neck cancer, cervical cancer, ovarian cancer, colorectal cancer, small intestine cancer, rectal cancer, fallopian tube carcinoma, perianal cancer, endometrial carcinoma, vaginal carcinoma, vulvar carcinoma, Hodgkin's disease, esophageal cancer, lymph adenocarcinoma, bladder cancer, gallbladder cancer, endocrine adenocarcinoma, thyroid cancer, parathyroid cancer, adrenal cancer, soft tissue sarcoma, urethral cancer, penile cancer, prostate cancer, chronic or acute leukemia, lymphocytic lymphoma, kidney or ureter cancer, renal cell carcinoma, renal pelvic carcinoma, central nervous system tumor, primary central nervous system lymphoma, spinal cord tumor, brainstem gliomas and pituitary adenomas, but is not limited thereto. Preferably, the cancer may be gastric cancer, but is not limited thereto.

As used herein, the term "prevention" refers to all actions that suppress the symptoms of a specific disease or delay its progression by administering the composition of the present disclosure.

As used herein, the term "treatment" refers to all actions that improve or beneficially change the symptoms of a specific disease by administering the composition of the present disclosure.

In one embodiment, the compound of the present disclosure, the hydrate thereof, the solvate thereof, or the pharmaceutically acceptable salt thereof may inhibit the proliferation of cancer cells.

In one embodiment, the compound of the present disclosure, the hydrate thereof, the solvate thereof, or the pharmaceutically acceptable salt thereof may inhibit PD-L1 expression in cancer cells.

The pharmaceutical composition of the present disclosure may be used as a single therapy for treating cancer, but may also be used in combination with other conventional biological therapy, chemotherapy or radiation therapy, and may more effectively treat cancer in the case of such combined therapy. In the case of using the present disclosure for preventing or treating cancer, the chemotherapeutic agent that may be used with the composition includes, for example, cisplatin, carboplatin, procarbazine, mechlorethamine, cyclophosphamide, ifosfamide, melphalan, chlorambucil, busulfan, nitrosourea, dactinomycin, daunorubicin, doxorubicin, bleomycin, plicomycin, mitomycin, etoposide, tamoxifen, taxol, transplatinum, 5-fluorouracil, vincristin, vinblastin, methotrexate, and the like.

In one aspect, the present disclosure provides a pharmaceutical composition for preventing or treating immune diseases, including the compound of the present disclosure, a hydrate thereof, a solvate thereof, or a pharmaceutically acceptable salt thereof.

In one embodiment, the immune disease may be at least any one selected from the group consisting of arthritis, inflammatory bowel disease, diabetes, degenerative neurological disease, psoriasis, systemic sclerosis, and transplant rejection disease, but is not limited thereto.

In one embodiment, the inflammatory bowel disease is not limited thereto, but may be at least any one selected from the group consisting of Crohn's disease, intestinal lesions accompanied with Behcet's disease, ulcerative colitis, hemorrhagic rectal ulcer, and pouchitis, and the composition of the present disclosure may have an effect of maintaining or restoring the colon length to a normal state.

In one embodiment, the compound of the present disclosure, the hydrate thereof, the solvate thereof, or the pharmaceutically acceptable salt thereof may inhibit inflammation.

In one embodiment, the compound of the present disclosure, the hydrate thereof, the solvate thereof, or the pharmaceutically acceptable salt thereof may increase the expression of Th1, Th2 and Th17 cells.

Unless otherwise stated, the 'treatment' means reversing, alleviating, inhibiting the progression of, or preventing the disease or disorder to which the term is applied, or one or more symptoms of the disease or disorder, and as used herein, the term 'treatment' refers to any treating action when defined as described above. Accordingly, treatment or therapy for immune diseases in mammals may include one or more of the following:
(1) inhibiting the growth of immune diseases, that is, arresting development thereof;
(2) preventing the spread of immune diseases;
(3) alleviating immune diseases;
(4) preventing the recurrence of immune diseases; and
(5) palliating the symptoms of immune diseases.

The pharmaceutical composition of the present disclosure may further include an adjuvant in addition to the active ingredient. The adjuvant may be used with any adjuvant known in the art without limitation, but further include, for example, a Freund's complete adjuvant or an incomplete adjuvant to increase the effect thereof.

The pharmaceutical composition according to the present disclosure may be prepared in the form of incorporating the active ingredient into a pharmaceutically acceptable carrier. Here, the pharmaceutically acceptable carrier includes carriers, excipients and diluents, which are commonly used in a pharmaceutical field. The pharmaceutically acceptable carrier that may be used in the pharmaceutical composition of the present disclosure is not limited thereto, but may include lactose, dextrose, sucrose, sorbitol, mannitol, xylitol, erythritol, maltitol, starch, acacia gum, alginate, gelatin, calcium phosphate, calcium silicate, cellulose, methyl cellulose, polyvinylpyrrolidone, water, methyl hydroxybenzoate, propyl hydroxybenzoate, talc, magnesium stearate, and mineral oil.

The pharmaceutical composition according to the present disclosure may be formulated and used in the form of oral formulations such as powders, granules, tablets, capsules, suspensions, emulsions, syrups, aerosols, etc., external preparations, suppositories, or sterile injectable solutions according to a conventional method.

The formulations may be prepared by using diluents or excipients, such as a filler, an extender, a binder, a wetting agent, a disintegrating agent, a surfactant, etc., which are generally used. Solid formulations for oral administration include tablets, pills, powders, granules, capsules, etc., and these solid formulations may be prepared by mixing at least one or more excipients, such as starch, calcium carbonate, sucrose, lactose, gelatin, etc. with the active ingredient. Further, lubricants such as magnesium stearate and talc may be used in addition to simple excipients. Liquid formulations for oral administration may correspond to suspensions, oral liquids, emulsions, syrups, etc., and may include various excipients, such as a wetting agent, a sweetener, an aromatic agent, a preserving agent, etc., in addition to commonly used diluents, such as water and liquid paraffin. Formulations for parenteral administration include sterile aqueous solutions, non-aqueous solvents, suspensions, emulsions, lyophilized agents, and suppositories. As the non-aqueous solvent and the suspension, propylene glycol, polyethylene glycol, vegetable oil such as olive oil, injectable ester such as ethyl oleate, etc. may be used. As a base material of the suppository, witepsol, Tween 61, cacao butter, laurinum, glycerogelatin, etc. may be used.

The pharmaceutical composition of the present disclosure may be administered to a subject through various routes. All methods of administration may be expected, and the pharmaceutical composition may be administered by, for example, oral, intravenous, intramuscular, subcutaneous, and intraperitoneal injection.

The dose of the pharmaceutical composition according to the present disclosure is selected in consideration of the age, body weight, sex, and physical condition of the subject. It is obvious that the concentration of the active ingredient included in the pharmaceutical composition may be variously selected according to a target, and preferably, the active ingredient is included in the pharmaceutical composition at a concentration of 0.01 to 5,000 µg/ml. When the concentration thereof is less than 0.01 µg/ml, pharmaceutical activity may not be exhibited, and when the concentration thereof is more than 5,000 µg/ml, toxicity to the human body may be exhibited.

In one aspect, the present disclosure provides a food composition for preventing or improving diseases associated with mitochondrial dysfunction, including the compound of the present disclosure, a hydrate thereof, a solvate thereof, or a pharmaceutically acceptable salt thereof.

In one aspect, the present disclosure provides a food composition for preventing or improving immune diseases, including the compound of the present disclosure, a hydrate thereof, a solvate thereof, or a pharmaceutically acceptable salt thereof.

In one aspect, the present disclosure provides a health functional food composition for preventing or improving diseases associated with mitochondrial dysfunction, including the compound of the present disclosure, a hydrate thereof, a solvate thereof, or a pharmaceutically acceptable salt thereof.

In one aspect, the present disclosure provides a health functional food composition for preventing or improving immune diseases, including the compound of the present disclosure, a hydrate thereof, a solvate thereof, or a pharmaceutically acceptable salt thereof.

The food composition of the present disclosure may contain various flavoring agents or natural carbohydrates as additional ingredients, like conventional food compositions, in addition to containing the compound of the present disclosure, the hydrate thereof, the solvate thereof, or the pharmaceutically acceptable salt thereof.

Examples of the above-described natural carbohydrates include conventional sugars, including monosaccharides, such as glucose, fructose, etc.; disaccharides, such as maltose, sucrose, etc.; and polysaccharides, such as dextrin, cyclodextrin, etc., and sugar alcohols such as xylitol, sorbitol, erythritol, etc. The above-described flavoring agents may be advantageously used with natural flavoring agents (thaumatin), stevia extracts (e.g., rebaudioside A, glycyrrhizin, etc.), and synthetic flavoring agents (saccharin, aspartame, etc.). The food composition of the present disclosure may be formulated in the same manner as the pharmaceutical composition to be used as a functional food or added to various foods. The food capable of adding the composition of the present disclosure includes, for example, beverages, meat, chocolate, foods, confectionery, pizza, ramen, other noodles, gums, candies, ice creams, alcoholic beverages, vitamin complexes, health food supplements, etc.

In addition, the food composition may contain various nutrients, vitamins, minerals (electrolytes), flavoring agents such as synthetic and natural flavoring agents, coloring agents and enhancers (cheese, chocolate, etc.), pectic acid and salts thereof, alginic acid and salts thereof, organic acid, a protective colloidal thickener, a pH adjusting agent, a stabilizer, a preservative, glycerin, alcohol, a carbonic acid agent used in a carbonated drink, and the like, in addition to the compound of the present disclosure, the hydrate thereof, the solvate thereof, or the pharmaceutically acceptable salt thereof as the active ingredient. In addition, the food composition of the present disclosure may contain natural fruit juice, and pulps for preparing fruit juice beverages, and vegetable beverages.

The functional food composition of the present disclosure may be prepared and processed in the form of tablets, capsules, powders, granules, liquids, pills, etc. for the purpose of prevention or treatment of diseases associated with mitochondrial dysfunction. As used herein, the 'health functional food composition' refers to food prepared and processed by using raw materials or ingredients with functionality, which are useful for the human body according to the Art No. 6727 on Health Functional Food, and means foods taken for adjusting nutrients for the structures and functions of the human body or obtaining a useful effect on health applications such as physiological actions. The health functional food of the present disclosure may include conventional food additives, and the suitability as the food additives is determined by the specifications and standards for the corresponding item in accordance with the general rules, general test methods, etc. of the Food Additives Codex, that are approved by the Food and Drug Administration, unless otherwise specified. The items disclosed in the 'Food Additives Codex' may include, for example, chemical composites such as ketones, glycine, calcium citrate, nicotinic acid, cinnamic acid, etc.; natural additives such as persimmon pigment, licorice extract, crystalline cellulose, Kaoliang color, guar gum, etc.; mixed formulations such as sodium L-glutamic acid formulations, noodle additive alkali agents, preservative formulations, tar color formulations, etc. For example, the health functional food in the form of tablets may be prepared by granulating a mixture obtained by mixing the active ingredient of the present disclosure with an excipient, a binder, a disintegrant, and other additives, and then compression-molding the mixture by adding a slip modifier and the like, or directly compression-molding the mixture. In addition, the health functional food in the form of tablets may also contain a flavor enhancer or the like as needed. In the health functional food in the form of capsules, hard capsules may be prepared by filling a mixture mixed with the active ingredient of the present disclosure and additives such as excipients into conventional hard capsules, and soft capsules may be prepared by filling a mixture mixed with the active ingredient of the present disclosure and additives such as excipients into capsule bases such as gelatin. The soft capsules may contain a plasticizer such as glycerin or sorbitol, a colorant, a preservative, and the like, if necessary. The health functional food in the form of pills may be prepared by molding a mixture obtained by mixing the active ingredient of the present disclosure with an excipient, a binder, a disintegrant, etc. by conventional known methods, and may also be coated with white sugar or other coating agents or surface-coated with materials such as starch and talc, if necessary. The health functional food in the form of granules may be prepared by granulizing a mixture obtained by mixing the active ingredient of the present disclosure with an excipient, a binder, a disintegrant, etc. by conventional known methods and may contain a flavoring agent, a flavos enhancer, etc., if necessary.

In one aspect, the present disclosure relates to a method for preparing a compound targeting mitochondria, including reacting (5-carboxypentyl)triphenylphosphonium bromide with carbonyldiimidazole; adding N-methyl-1,3-propanediamine dropwise and mixing the mixture; and reacting with dicyandiamide.

The preparing method of the present disclosure has the advantages of having fewer steps, taking less time, and having a significantly high yield.

In one aspect, the present disclosure relates to nanoparticles including a compound represented by the following Chemical Formula 1, a hydrate thereof, a solvate thereof, or a pharmaceutically acceptable salt thereof:

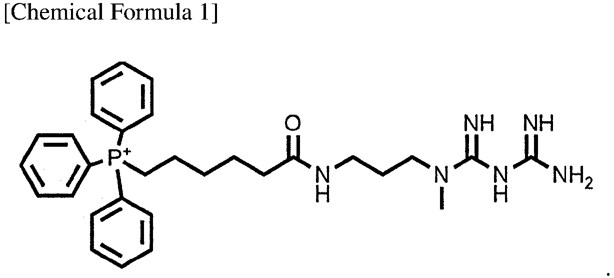

The "nanoparticle" of the present disclosure refers to a microparticle whose particle size may be expressed in nanometer units, and the nanoparticles of the present disclosure may be made of any one material selected from the group consisting of noble metals such as gold (Au), silver (Ag), platinum (Pt), palladium (Pd), iridium (Ir), rhodium (Rh), and ruthenium (Ru); titanium (Ti), zirconium (Zr), hafnium (Hf), vanadium (V), niobium (Nb), tantalum (Ta), chromium (Cr), molybdenum (Mo), tungsten (W), ruthenium (Ru), and osmium (Os); iron (Fe), nickel (Ni), and cobalt (Co); magnesium oxide (MgO), titanium dioxide (TiO₂), vanadium pentoxide (V₂O₅), zinc oxide (ZnO), latex beads, magnetic beads, quantum dots, upconversion nanoparticles (UCNP), graphene-nanoparticle composites, ceramics, polymers, semiconductors, perovskites, plastics, carbon dots, non-metals, and color dyed particles, but are not limited thereto. In one embodiment, the nanoparticles may preferably be gold nanoparticles.

In one embodiment, the compound represented by Chemical Formula 1, the hydrate thereof, the solvate thereof, or the pharmaceutically acceptable salt thereof is characterized to be bound to the surface of the nanoparticles.

In one embodiment, the size of the nanoparticle may be 1 to 200 nm, but is not limited thereto. The size of the nanoparticle may be preferably 1 to 150 nm, and more preferably 1 to 100 nm, but is not limited thereto.

In one embodiment, the compound represented by Chemical Formula 1, the hydrate thereof, the solvate thereof, or the pharmaceutically acceptable salt thereof may be included in an amount of 200 to 400 nmol per 1 nmol of nanoparticles, but is not limited thereto. The compound represented by Chemical Formula 1, the hydrate thereof, the solvate thereof, or the pharmaceutically acceptable salt thereof may be included in an amount of preferably 300 to 400 nmol, and more preferably 310 to 320 nmol, but is not limited thereto.

In one aspect, the present disclosure relates to a pharmaceutical composition for preventing or treating diseases associated with mitochondrial dysfunction, including nanoparticles according to the present disclosure.

In one embodiment, the disease associated with mitochondrial dysfunction may be at least any one selected from the group consisting of cancer disease, ischemic brain disease, ischemic heart disease, stroke, migraine, anemia, Parkinson's disease, Alzheimer's disease, amyotrophic lateral sclerosis (ALS), mitochondrial encephalomyopathy, liver cirrhosis, Fanconi syndrome, atypical phenylketonuria, asthma, diabetes, arteriosclerosis, hypertension, hypercholesterolemia, optic nerve disease, obesity, and depression, but is not limited thereto.

In one aspect, the present disclosure relates to a pharmaceutical composition for preventing or treating immune diseases, including nanoparticles according to the present disclosure.

In one embodiment, the immune disease may be at least any one selected from the group consisting of arthritis, inflammatory bowel disease, diabetes, degenerative neurological disease, psoriasis, systemic sclerosis, and transplant rejection disease, but is not limited thereto.

In one aspect, the present disclosure relates to a food composition for preventing or improving diseases associated with mitochondrial dysfunction, including nanoparticles according to the present disclosure.

In one aspect, the present disclosure relates to a food composition for preventing or improving immune diseases, including nanoparticles according to the present disclosure.

In one aspect, the present disclosure relates to a health functional food composition for preventing or improving diseases associated with mitochondrial dysfunction, including nanoparticles according to the present disclosure.

In one aspect, the present disclosure relates to a health functional food composition for preventing or improving immune diseases, including nanoparticles according to the present disclosure.

The specific description of the pharmaceutical composition, the food composition or the health functional food composition including the nanoparticles of the present disclosure is the same as described above.

In one aspect, the present disclosure relates to a method for preventing or treating diseases associated with mitochondrial dysfunction, including administering, to a subject, the compound of the present disclosure, a hydrate thereof, a solvate thereof, or a pharmaceutically acceptable salt thereof.

In one aspect, the present disclosure relates to a method for preventing or treating diseases associated with mitochondrial dysfunction, including administering, to a subject, nanoparticles including the compound of the present disclosure, a hydrate thereof, a solvate thereof, or a pharmaceutically acceptable salt thereof.

In one aspect, the present disclosure relates to a method for preventing or treating immune diseases, including administering, to a subject, the compound of the present disclosure, a hydrate thereof, a solvate thereof, or a pharmaceutically acceptable salt thereof.

In one aspect, the present disclosure relates to a method for preventing or treating immune diseases, including administering, to a subject, nanoparticles including the compound of the present disclosure, a hydrate thereof, a solvate thereof, or a pharmaceutically acceptable salt thereof.

The subject is preferably mammals, including humans, and includes all patients who are undergoing treatment, have received treatment, and need to receive treatment for at least one disease selected from the group consisting of diseases associated with mitochondrial dysfunction and immune diseases. In addition, the subject may include patients who have undergone a surgical operation for the treatment of at least one disease selected from the group consisting of diseases associated with mitochondrial dysfunction and immune diseases.

In one embodiment, the disease associated with mitochondrial dysfunction may be at least any one selected from the group consisting of cancer disease, ischemic brain disease, ischemic heart disease, stroke, migraine, anemia, Parkinson's disease, Alzheimer's disease, amyotrophic lateral sclerosis (ALS), mitochondrial encephalomyopathy, liver cirrhosis, Fanconi syndrome, atypical phenylketonuria, asthma, diabetes, arteriosclerosis, hypertension, hypercholesterolemia, optic nerve disease, obesity, and depression, but is not limited thereto.

In an embodiment, the cancer may be cancer caused by mitochondrial hypofunction, and may be at least any one selected from the group consisting of gastric cancer, brain tumor, melanoma, myeloma, non-small cell lung cancer, oral cancer, liver cancer, colon cancer, breast cancer, lung cancer, bone cancer, pancreatic cancer, skin cancer, head or neck cancer, cervical cancer, ovarian cancer, colorectal cancer, small intestine cancer, rectal cancer, fallopian tube carcinoma, perianal cancer, endometrial carcinoma, vaginal carcinoma, vulvar carcinoma, Hodgkin's disease, esophageal cancer, lymph adenocarcinoma, bladder cancer, gallbladder cancer, endocrine adenocarcinoma, thyroid cancer, parathyroid cancer, adrenal cancer, soft tissue sarcoma, urethral cancer, penile cancer, prostate cancer, chronic or acute leukemia, lymphocytic lymphoma, kidney or ureter cancer, renal cell carcinoma, renal pelvic carcinoma, central nervous system tumor, primary central nervous system lymphoma, spinal cord tumor, brainstem gliomas and pituitary adenomas, but is not limited thereto. Preferably, the cancer may be gastric cancer, but is not limited thereto.

In one embodiment, the immune disease may be at least any one selected from the group consisting of arthritis, inflammatory bowel disease, diabetes, degenerative neurological disease, psoriasis, systemic sclerosis, and transplant rejection disease, but is not limited thereto.

Hereinafter, the present disclosure will be described in more detail through the following Examples. However, the following Examples are only intended to embody the contents of the present disclosure, and the present disclosure is not limited thereto.

### Example 1. Synthesis of novel compounds targeting mitochondria

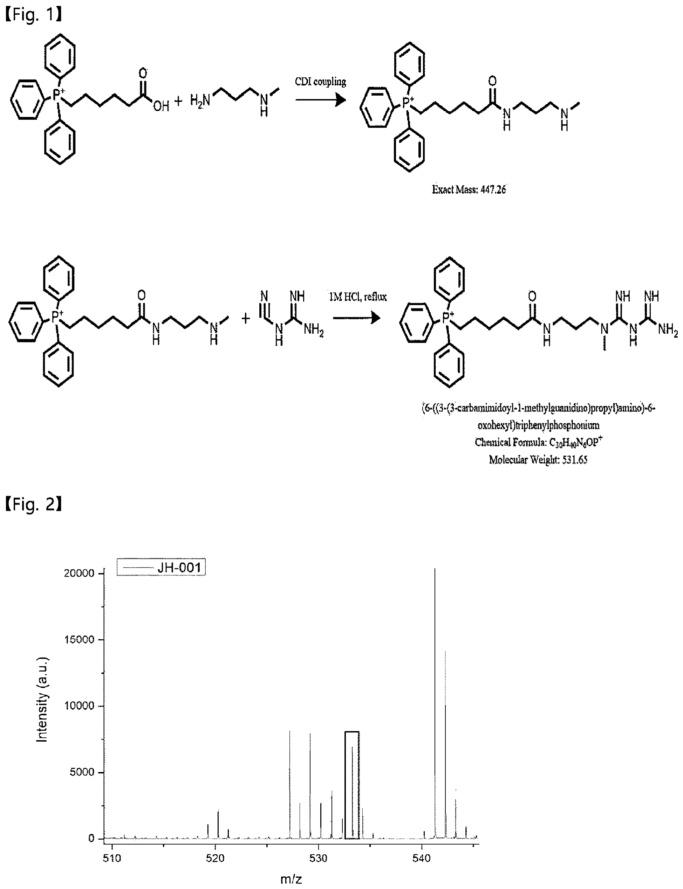

To enhance a mitochondria-targeting function, compounds containing a mitochondria-targeting moiety were synthesized. Specifically, 1.37 g (3 mmol) of (5-carboxypentyl)triphenylphosphonium bromide and 535.1 mg (3.3 mmol) of carbonyldiimidazole were dissolved in 10 ml of dichloromethane, reacted for 15 minutes, and a temperature was lowered in an ice bath to obtain a compound of Chemical Formula 2. Thereafter, 277.6 g (3.15 mmol) of N-methyl-1,3-propanediamine dissolved in 5 ml of dichloromethane was slowly added dropwise to the compound of Chemical Formula 2, mixed, and then reacted for 2 hours, and a solvent was removed using an evaporator to obtain a compound of Chemical Formula 3 (mass: 447.26). The compound of Chemical Formula 3 was dissolved in 20 ml of 1 N HCl, added with 3 g (36 mmol) of dicyandiamide dissolved in 10 ml of 1N HCl, and then reacted overnight at 100°C to obtain a mitochondria-targeting compound of Chemical Formula 1 ((6-((3-(3-carbamimidoyl-1-methylguanidino)propyl)amino)-6-oxohexyl)triphenylphosphonium, C₃₀H₄₀N₆OP⁻, molecular weight: 531.65) (referred to as JH-001) with yield of about 80 to 85% based on a starting material (FIG. 1).

### Example 2. Mass analysis of mitochondria-targeting compounds

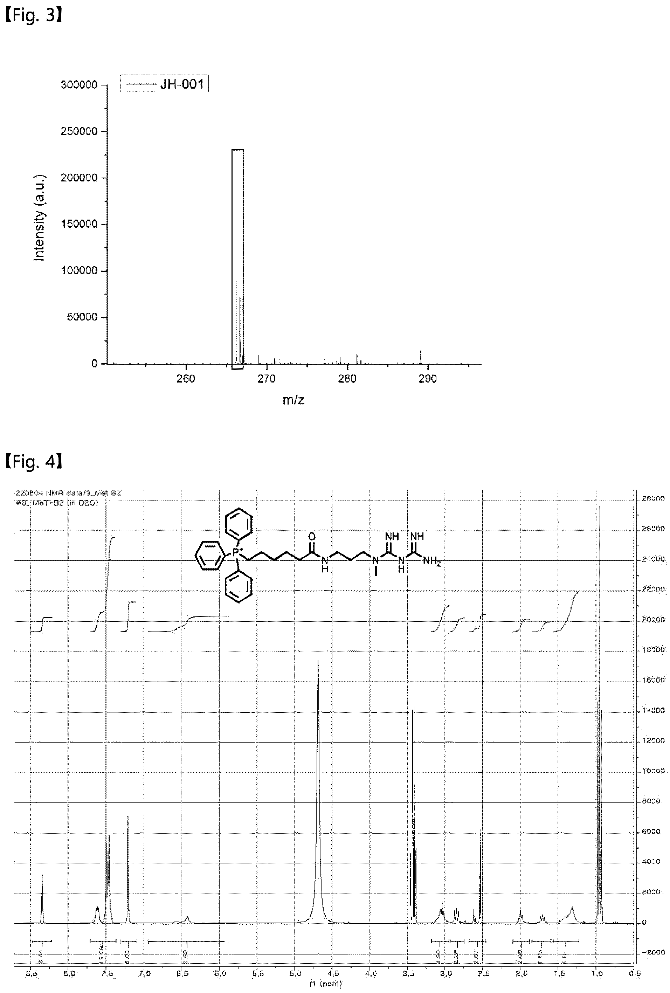

As a result of performing mass analysis of the mitochondria-targeting compounds synthesized in Example 1 using an electron sprayed ionization mass spectrometer, it was confirmed that one hydrogen was added to the synthesized compound to show a peak corresponding to 532.65 (FIG. 2). In addition, even in the result analyzed in a different range, it was confirmed that one hydrogen was added to the synthesized compound and two charges were contained to show a peak corresponding to 532/2 = 266 (FIG. 3), so that a mitochondria-targeting compound ((6-((3-(3-carbamimidoyl-1-methylguanidino)propyl)amino)-6-oxohexyl)triphenylphosphonium, C₃₀H₄₀N₆OP⁻) with a molecular weight of 531.65 was synthesized.

### Example 3. Nuclear magnetic resonance analysis of mitochondria-targeting compounds

As a result of confirming the structure of the mitochondria-targeting compound synthesized in Example 1 by nuclear magnetic resonance, it was confirmed that (6-((3-(3-carbamimidoyl-1-methylguanidino)propyl)amino)-6-oxohexyl)triphenylphosphonium of Chemical Formula 1 was synthesized (FIG. 4).
¹H 300 MHz, ppm: 1.2-1.5 (m, 6H), 1.7 (t, 2H), 2.0 (t, 2H) 2.52 (s, 3H), 2.7 (t, 2H), 3.1 (t, 2H), 3.2 (br, 2H), 7.2 (s, 4H) 7.9-8.0 (s, 15H) 8.1 (br, 1H)

### Example 4. Confirmation of effect of inhibiting mitochondria-targeting compounds on gastric cancer cell proliferation

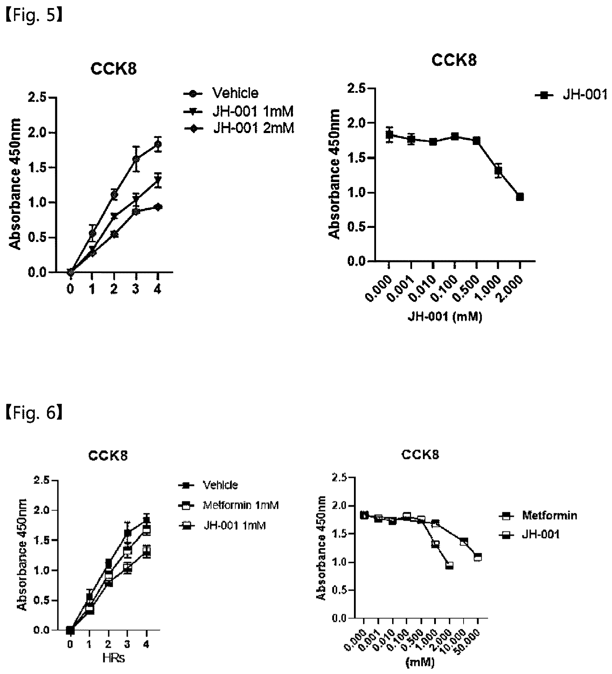

After treating an AGS cell line, a gastric cancer cell line, with a mitochondria-targeting compound JH-001 for each concentration, a CCK8 assay was performed after 48 hours to confirm whether JH-001 inhibited gastric cancer cell proliferation. As a result, it was shown that the proliferation of gastric cancer cells was inhibited in a concentration-dependent manner by JH-001 (FIG. 5). In addition, CCK-8 analysis was performed on cell activity by treating JH-001 and metformin (Sigma Aldrich, Cat No# PHR1084), a conventional drug known as a mitochondria-targeting drug at 1 mM concentrations, respectively. As a result, JH-001 showed a higher cell death effect depending on the treatment time compared to metformin at the same concentration (FIG. 6). In addition, JH-001 showed a similar effect to the known drug, metformin at a low concentration, but showed a significantly better cell killing effect in a concentration-dependent manner.

### Example 5. Confirmation of PD-L1 inhibition effect of mitochondria-targeting compounds

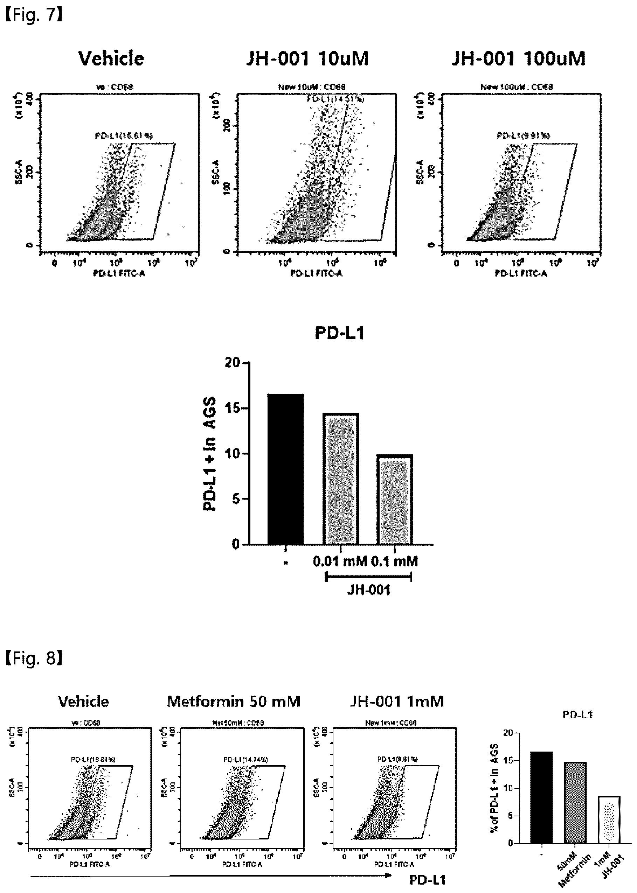

After treating an AGS cell line, a gastric cancer cell line, with JH-001 for each concentration, the expression of PD-L1, an immunocompromised marker, was confirmed using a flow cytometer. As a result, it was found that the expression of PD-L1 in gastric cancer cells was inhibited in a concentration-dependent manner of JH-001 (FIG. 7). In addition, metformin, known as a mitochondria-targeting drug, was treated at a concentration 50 times higher than that of JH-001 and the expression level of PD-L1 was confirmed. As a result, it was confirmed that JH-001 according to the present disclosure inhibited PD-L1 expression about 2 times higher than metformin treatment, even though JH-001 was treated at a much lower concentration (FIG. 8).

### Example 6. Confirmation of effect of mitochondria-targeting compounds on treating inflammatory bowel disease

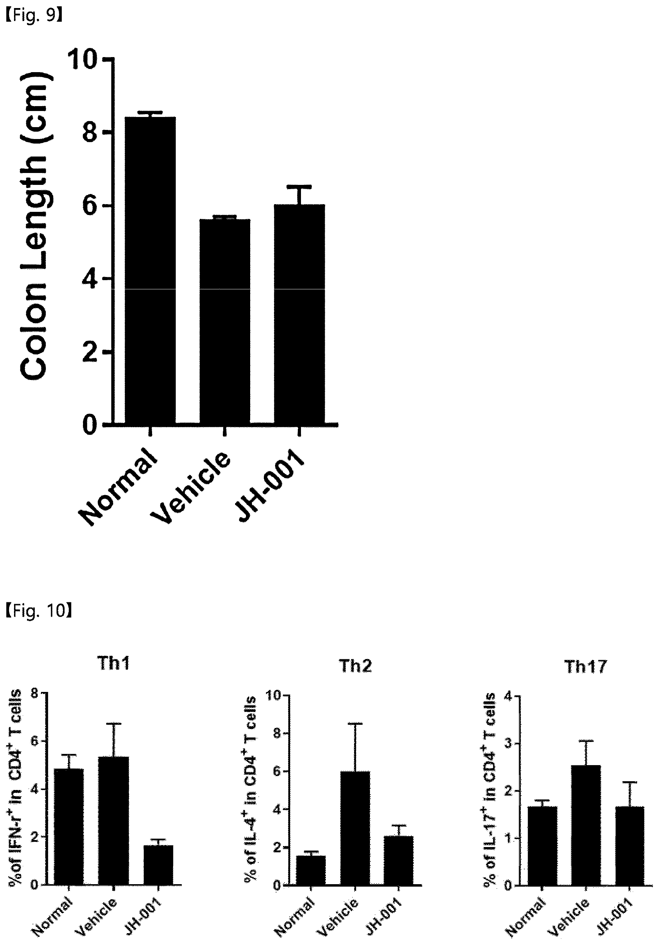

C57BL/6 mice (male, 8 weeks old, Orient Bio) were orally administered with 3% dextran sulfate sodium (DSS) for 5 days to induce colitis, and DSS-induced mice as an inflammatory bowel disease model were fabricated, and then orally administered with JH-001 daily at 10 mg/kg to be euthanized, and then the colon length was measured. As a result, it was shown that the colon length was recovered compared to a control group (vehicle) when JH-001 was administered (FIG. 9).

### Example 7. Confirmation of inflammation inhibitory effect of mitochondria-targeting compounds

Spleens were extracted from DSS-induced mice as an inflammatory bowel disease model, and the expression of Th1 (CD4+, IFN-r+), Th2 (CD4+, IL-4+), and Th17 (CD4+, IL-17+) cells was analyzed using a flow cytometer.

As a result, it was found that the expression levels of Th1, Th2, and Th17 were decreased compared to the control group (vehicle) when JH-001 was administered (FIG. 10), so that it was confirmed that inflammatory cells were inhibited by JH-001.

### Example 8. Fabrication of gold nanoparticles containing mitochondria-targeting compound

To confirm an effect of Example 7 on systemic sclerosis in a mouse model, a gold nanoparticle-mitochondria-targeting compound was synthesized. Gold nanoparticle synthesis was performed by referring to a method of Piella et al. (Jordi Piella, et al. Chem. Mater. 2016, 28, 1066-1075.). 2.2 mM sodium citrate (SC) was dissolved in 150 ml of tertiary distilled water and mixed with 0.1 ml of 2.5 mM tannic acid (TA) and 1 ml of 150 mM potassium carbonate (PC). After heating to 70°C, tetrachloroauric acid (25 mM) dissolved in 1 ml of tertiary distilled water was quickly added and reacted for 10 minutes. Thereafter, after cooling to room temperature, the formed gold nanoparticles were centrifuged at 2500 rpm for 5 minutes using a centrifugal filter (30 kDa MWCO) to remove unreacted SC, TA, and PC. Thereafter, a surface substitution experiment was performed by slowly adding 200 times the mole number of methoxy polyethylene glycol-thiol (mPEG-SH) compared to that of the gold nanoparticles and reacting for 15 minutes. Thereafter, the unreacted excess mPEG-SH was removed by centrifugation at 2500 rpm for 5 minutes using a centrifugal filter (30 kDa MWCO). The obtained gold nanoparticle solution was then slowly added to 0.7 mM JH-001 and reacted at room temperature for 15 minutes. After the reaction was finished, unreacted JH-001 was dialyzed by centrifugation at 2500 rpm for 5 minutes using a centrifugal filter (30 kDa MWCO). As a final result, gold nanoparticles containing JH-001 were fabricated. The gold nanoparticles containing JH-001 were fabricated in a structure in which JH-001 was bound to the surface of the gold nanoparticles and inserted between surface ligands. In the fabricated gold nanoparticles, the absorbance was measured to confirm the optical characteristics according to a method of Piella et al.

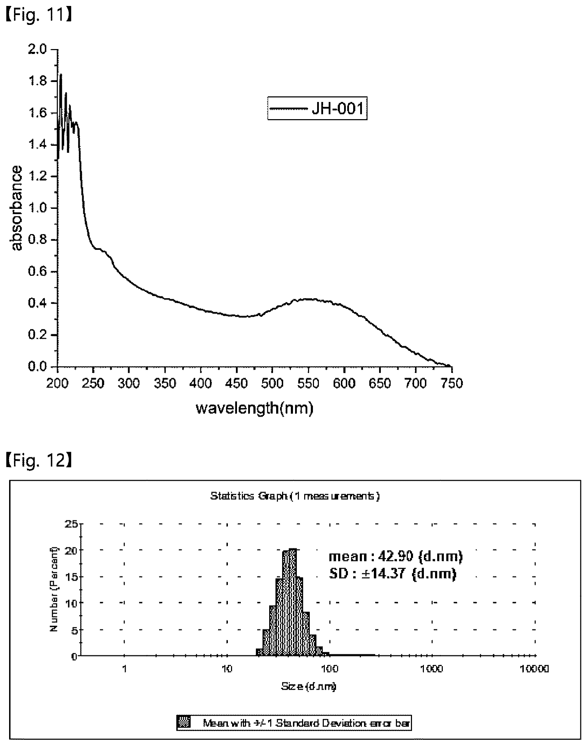

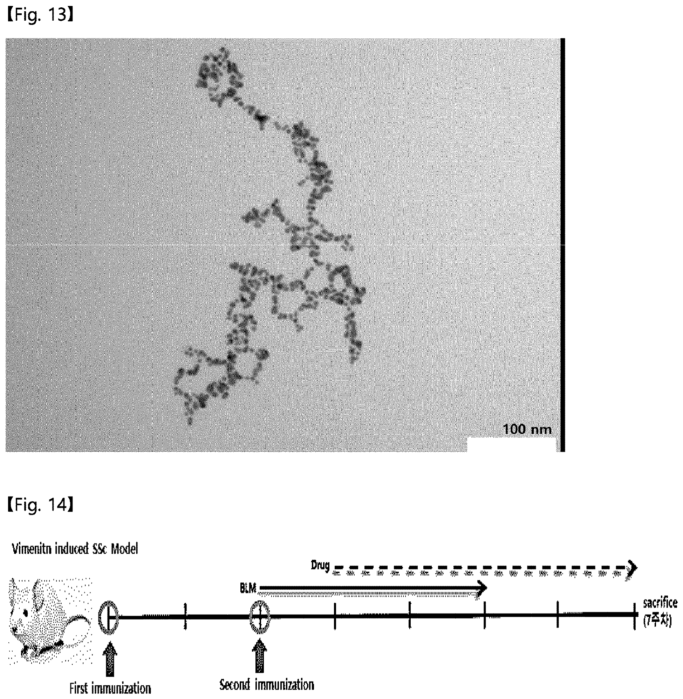

As a result, the absorbance of gold nanoparticles containing JH-001 was confirmed (FIG. 11). The absorbance of JH-001 at 267 nm was confirmed as 0.725, which was confirmed that 0.168 mg (316 nmol) of JH-001 was bound to the surface of gold nanoparticles per 1 nmol of nanoparticles. In addition, as a result of confirming a hydrated size distribution histogram and transmission electron microscopy of the obtained gold nanoparticles containing JH-001, it was confirmed to have a particle size distribution of 1 to 100 nm (FIGS. 12 and 13).

### Example 9. Confirmation of regulation of vimentin autoantibody expression by gold nanoparticles containing mitochondria-targeting compound

In order to confirm a therapeutic effect of the gold nanoparticles containing JH-001 of Example 8 on systemic sclerosis (SSc), a vimentin-sensitized systemic sclerosis animal model was fabricated. Specifically, a vimentin protein was immunized into the nuchal region of an 8-week-old SKG mouse under subcutaneous injection (SC) at week 0 and week 2. Thereafter, each 100 µL of bleomycin (BLM) at a concentration of 0.5 mg/mL was injected subcutaneously for 21 days. Thereafter, from week 3 of systemic sclerosis induction, the gold nanoparticles containing JH-001 were subcutaneously injected twice a week at a concentration of 0.5 mg/kg (FIG. 14). After seven weeks of systemic sclerosis induction, mice in each group were humanely sacrificed, and serum from the sacrificed mice was obtained and then vimentin antigen-specific IgG and IgG1 were confirmed through ELISA. As a control group, an SSc-V group that was not treated with the gold nanoparticles containing JH-001 after induction of systemic sclerosis was used.

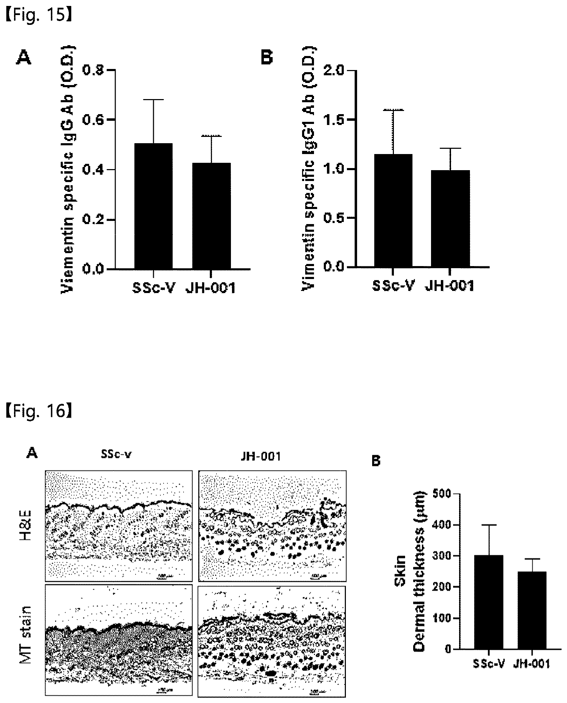

As a result, compared to the SSc-V group, it was confirmed that IgG and IgG1 were reduced in a JH-001 group (FIGS. 15A and 15B). Therefore, it was confirmed that gold nanoparticles containing JH-001 reduced a vimentin-specific autoantigen *in vivo.*

### Example 10. Confirmation of inhibition of systemic sclerosis by gold nanoparticles containing mitochondria-targeting compound

In addition, inflammatory and fibrotic responses in the skin tissue of each experimental group were analyzed histologically. Specifically, skin tissues were obtained from mice of each experimental group sacrificed in Example 9 above, and then the thickness of the dermal layer was confirmed through Hematoxylin & Eosin (H&E) staining. In addition, the degree of skin fibrosis, which was a symptom of systemic sclerosis, was determined by confirming collagen accumulation in skin tissue through Masson's trichrome (MT) staining. As a control group, an SSc-V group that was not treated with the gold nanoparticles containing JH-001 after induction of systemic sclerosis was used.

As a result, it was confirmed that compared to the SSc-V group, the thickness of the dermal layer of the skin was reduced in the JH-001 group, and collagen accumulation was also significantly inhibited. Therefore, it was confirmed that the gold nanoparticles containing JH-001 improved dermal sclerosis *in vivo* (FIGS. 16A and 16B).

## Claims

1. A compound represented by the following Chemical Formula 1, a hydrate thereof, a solvate thereof, or a pharmaceutically acceptable salt thereof:

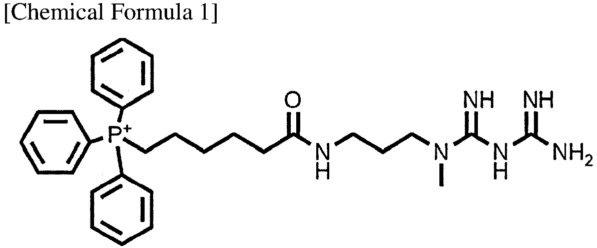

2. The compound represented by Chemical Formula 1, the hydrate thereof, the solvate thereof, or the pharmaceutically acceptable salt thereof of claim 1, wherein the compound represented by Chemical Formula 1 targets mitochondria.

3. The compound represented by Chemical Formula 1, the hydrate thereof, the solvate thereof, or the pharmaceutically acceptable salt thereof of claim 1, wherein the compound represented by Chemical Formula 1 further includes a labeling material.

4. The compound represented by Chemical Formula 1, the hydrate thereof, the solvate thereof, or the pharmaceutically acceptable salt thereof of claim 3, wherein the labeling material is at least one selected from the group consisting of a chromogenic enzyme, a radioisotope, a chromophore, a chromogenic material, a luminescent material, a fluorescer, superparamagnetic particles, and ultrasuper paramagnetic particles.

5. The compound represented by Chemical Formula 1, the hydrate thereof, the solvate thereof, or the pharmaceutically acceptable salt thereof of claim 4, wherein the fluorescent material is at least one selected from the group consisting of a fluorescent protein, a photoprotein, luciferase, a fluorescent dye, and time-resolved fluorescence (TRF).

6. A mitochondria-targeting composition comprising the compound of claim 1, a hydrate thereof, a solvate thereof, or a pharmaceutically acceptable salt thereof.

7. A pharmaceutical composition for preventing or treating diseases associated with mitochondrial dysfunction, comprising the compound of claim 1, a hydrate thereof, a solvate thereof, or a pharmaceutically acceptable salt thereof.

8. The pharmaceutical composition for preventing or treating diseases associated with mitochondrial dysfunction of claim 7, wherein the disease associated with mitochondrial dysfunction is at least one selected from the group consisting of cancer disease, ischemic brain disease, ischemic heart disease, stroke, migraine, anemia, Parkinson's disease, Alzheimer's disease, amyotrophic lateral sclerosis (ALS), mitochondrial encephalomyopathy, liver cirrhosis, Fanconi syndrome, atypical phenylketonuria, asthma, diabetes, arteriosclerosis, hypertension, hypercholesterolemia, optic nerve disease, obesity, and depression.

9. The pharmaceutical composition for preventing or treating diseases associated with mitochondrial dysfunction of claim 7, wherein the cancer is cancer caused by mitochondrial hypofunction.

10. The pharmaceutical composition for preventing or treating diseases associated with mitochondrial dysfunction of claim 7, wherein the cancer is at least one selected from the group consisting of gastric cancer, brain tumor, melanoma, myeloma, non-small cell lung cancer, oral cancer, liver cancer, colon cancer, breast cancer, lung cancer, bone cancer, pancreatic cancer, skin cancer, head or neck cancer, cervical cancer, ovarian cancer, colorectal cancer, small intestine cancer, rectal cancer, fallopian tube carcinoma, perianal cancer, endometrial carcinoma, vaginal carcinoma, vulvar carcinoma, Hodgkin's disease, esophageal cancer, lymph adenocarcinoma, bladder cancer, gallbladder cancer, endocrine adenocarcinoma, thyroid cancer, parathyroid cancer, adrenal cancer, soft tissue sarcoma, urethral cancer, penile cancer, prostate cancer, chronic or acute leukemia, lymphocytic lymphoma, kidney or ureter cancer, renal cell carcinoma, renal pelvic carcinoma, central nervous system tumor, primary central nervous system lymphoma, spinal cord tumor, brainstem gliomas and pituitary adenomas.

11. The pharmaceutical composition for preventing or treating diseases associated with mitochondrial dysfunction of claim 7, wherein the compound of claim 1, the hydrate thereof, the solvate thereof, or the pharmaceutically acceptable salt thereof inhibits the proliferation of cancer cells.

12. The pharmaceutical composition for preventing or treating diseases associated with mitochondrial dysfunction of claim 7, wherein the compound of claim 1, the hydrate thereof, the solvate thereof, or the pharmaceutically acceptable salt thereof inhibits the PD-L1 expression in cancer cells.

13. A food composition for preventing or improving diseases associated with mitochondrial dysfunction, comprising the compound of claim 1, a hydrate thereof, a solvate thereof, or a pharmaceutically acceptable salt thereof.

14. A health functional food composition for preventing or improving diseases associated with mitochondrial dysfunction, comprising the compound of claim 1, a hydrate thereof, a solvate thereof, or a pharmaceutically acceptable salt thereof.

15. A pharmaceutical composition for preventing or treating immune diseases, comprising the compound of claim 1, a hydrate thereof, a solvate thereof, or a pharmaceutically acceptable salt thereof.

16. The pharmaceutical composition for preventing or treating immune diseases of claim 15, wherein the immune disease is at least one selected from the group consisting of arthritis, inflammatory bowel disease, diabetes, degenerative neurological disease, psoriasis, systemic sclerosis, and transplant rejection disease.

17. The pharmaceutical composition for preventing or treating immune diseases of claim 15, wherein the compound of claim 1, the hydrate thereof, the solvate thereof, or the pharmaceutically acceptable salt thereof inhibits inflammation.

18. The pharmaceutical composition for preventing or treating immune diseases of claim 15, wherein the compound of claim 1, the hydrate thereof, the solvate thereof, or the pharmaceutically acceptable salt thereof increases the expression of Th1, Th2 and Th17 cells.

19. A food composition for preventing or improving immune diseases, comprising the compound of claim 1, a hydrate thereof, a solvate thereof, or a pharmaceutically acceptable salt thereof.

20. A health functional food composition for preventing or improving immune diseases, comprising the compound of claim 1, a hydrate thereof, a solvate thereof, or a pharmaceutically acceptable salt thereof.

21. A method for preparing a compound targeting mitochondria, comprising:
a) reacting (5-carboxypentyl)triphenylphosphonium bromide with carbonyldiimidazole;
b) adding N-methyl-1,3-propanediamine dropwise and mixing the mixture; and
c) reacting with dicyandiamide.

22. A nanoparticle comprising a compound represented by the following Chemical Formula 1, a hydrate thereof, a solvate thereof, or a pharmaceutically acceptable salt thereof:

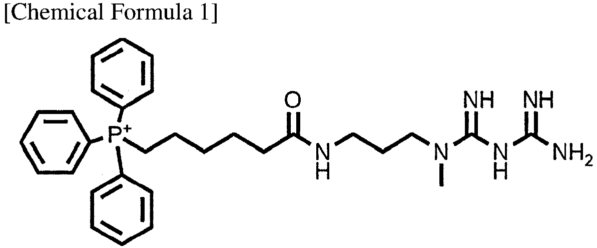

23. The nanoparticle of claim 22, wherein the nanoparticle is made of any one selected from the group consisting of noble metals of gold (Au), silver (Ag), platinum (Pt), palladium (Pd), iridium (Ir), rhodium (Rh), and ruthenium (Ru); Titanium (Ti), zirconium (Zr), hafnium (Hf), vanadium (V), niobium (Nb), tantalum (Ta), chromium (Cr), molybdenum (Mo), tungsten (W), ruthenium (Ru), osmium (Os); iron (Fe), nickel (Ni), cobalt (Co); magnesium oxide (MgO), titanium dioxide (TiO₂), vanadium pentoxide (V₂O₅), zinc oxide (ZnO), latex beads, magnetic beads, quantum dots, upconversion nanoparticles (UCNP), a graphene-nanoparticle complex, ceramics, polymers, semiconductors, perovskites, plastics, carbon dots, non-metals, and color dyed particles.

24. The nanoparticle of claim 22, wherein the compound represented by Chemical Formula 1, the hydrate thereof, the solvate thereof, or the pharmaceutically acceptable salt thereof is included in an amount of 200 to 400 nmol per 1 nmol of nanoparticles.

25. A pharmaceutical composition for preventing or treating diseases associated with mitochondrial dysfunction, comprising the nanoparticles of claim 22.

26. The pharmaceutical composition for preventing or treating diseases associated with mitochondrial dysfunction of claim 25, wherein the disease associated with mitochondrial dysfunction is at least one selected from the group consisting of cancer disease, ischemic brain disease, ischemic heart disease, stroke, migraine, anemia, Parkinson's disease, Alzheimer's disease, amyotrophic lateral sclerosis (ALS), mitochondrial encephalomyopathy, liver cirrhosis, Fanconi syndrome, atypical phenylketonuria, asthma, diabetes, arteriosclerosis, hypertension, hypercholesterolemia, optic nerve disease, obesity, and depression.

27. A food composition for preventing or improving diseases associated with mitochondrial dysfunction, comprising the nanoparticles of claim 22.

28. A health functional food composition for preventing or improving diseases associated with mitochondrial dysfunction, comprising the nanoparticles of claim 22.

29. A pharmaceutical composition for preventing or treating immune diseases, comprising nanoparticles of claim 22.

30. The pharmaceutical composition for preventing or treating immune diseases of claim 29, wherein the immune disease is at least one selected from the group consisting of arthritis, inflammatory bowel disease, diabetes, degenerative neurological disease, psoriasis, systemic sclerosis, and transplant rejection disease.

31. A food composition for preventing or improving immune diseases, comprising nanoparticles of claim 22.

32. A health functional food composition for preventing or improving immune diseases, comprising nanoparticles of claim 22.

33. A method for preventing or treating diseases associated with mitochondrial dysfunction, comprising administering, to a subject, a compound represented by the following Chemical Formula 1, a hydrate thereof, a solvate thereof, or a pharmaceutically acceptable salt thereof.

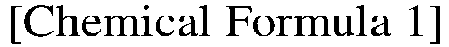

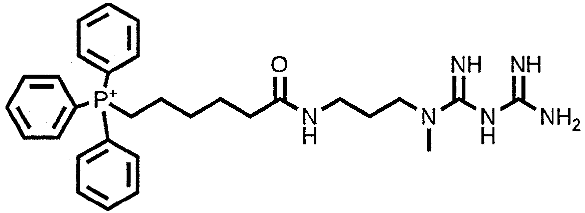

34. A method for preventing or treating immune diseases, comprising administering, to a subject, the compound of claim 1, a hydrate thereof, a solvate thereof, or a pharmaceutically acceptable salt thereof.

35. A method for preventing or treating diseases associated with mitochondrial dysfunction, comprising administering, to a subject, nanoparticles including a compound represented by the following Chemical Formula 1, a hydrate thereof, a solvate thereof, or a pharmaceutically acceptable salt thereof.

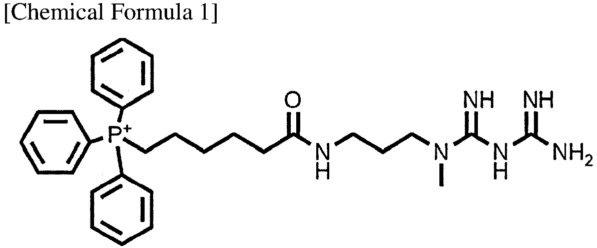

36. A method for preventing or treating immune diseases, comprising administering, to a subject, nanoparticles including the compound of claim 1, a hydrate thereof, a solvate thereof, or a pharmaceutically acceptable salt thereof.
